# EUROPÄISCHE PATENTSCHRIFT

(11) **EP 0 971 743 B1**
(45) Veröffentlichungstag und Bekanntmachung des Hinweises auf die Patenterteilung: **12.07.2006**
(21) Anmeldenummer: 97919382.8
(22) Anmeldetag: 18.04.1997
(51) Int. Cl.: A61K 45/06, A61K 31/195, A61K 31/375, A61K 38/06, A61K 31/015, A61K 31/355, A61K 47/00

(54) **STABILISIERTES ARZNEIMITTEL ENTHALTEND CYSTEINYLDERIVATE**
STABILIZED MEDICAMENTS CONTAINING CYSTEINYL DERIVATIVES
MEDICAMENTS STABILISES RENFERMANT DES DERIVES CYSTEINYLE

(43) Veröffentlichungstag der Anmeldung: 19.01.2000
(73) Patentinhaber: Stanislaus, Fritz, 81673 München (DE)
(72) Erfinder: Stanislaus, Fritz, 81673 München (DE)
(74) Vertreter: HOFFMANN EITLE
(86) Internationale Anmeldenummer: PCT/EP1997/001941
(87) Internationale Veröffentlichungsnummer: WO 1998/047534

(56) Entgegenhaltungen:
- EP-A- 0 511 895
- EP-A- 0 595 766
- WO-A-91/11117
- WO-A-92/21368
- WO-A-94/02036
- WO-A-96/10402
- DE-A- 3 542 309
- DE-U- 29 707 005
- US-A- 5 156 852
- US-A- 5 629 023
- CHEN, TAPPEL: "Protection of Multiple Antioxidants against Heme Protein Oxidation and Lpid Peroxidation Induced by CBrCl3 in Liver, Lung, Kidney, Heart and Spleen" J. AGRIC. FOOD CHEM., Bd. 44, Nr. 3, 1996, Seiten 854-854-858, XP002051329

## Beschreibung

Die Erfindung betrifft Arzneimittel mit einem Gehalt an Cysteinderivaten, die eine verbesserte Stabilität aufweisen.

Arzneistoffe mit einer Cysteinylgruppe stellen bedeutsame Vertreter des Arzneischatzes dar. Zu deren wichtigsten Verbindungen gehört das N-Acetylcystein, das bei verschiedenen Erkrankungen der Lunge und der Bronchien eine mukolytische Wirkung zeigt und deshalb bei deren Erkrankungen, z.B. Erkältungen und entzündeten Bronchien und gewissen asthmatischen Zuständen besonders als Sekretolytikum häufig verordnet wird. Daneben haben auch andere Cysteinderivate, wie das S-(Carboxymethyl)-cystein oder das N-Acetyl-3-[2-benzoylpropyl)]-thioalanin (Bencistein) bei den vorgenannten Indikationen eine gewisse Bedeutung als Sekretolytika erlangt.

Eine andere wichtige Gruppe therapeutisch eingesetzter Cysteinderivate stellen vor allem sogenannte Detoxikationsmittel oder Detoxicantien dar, die als physiologische Entgiftungsmittel oder auch Leberschutzmittel eingesetzt werden. Darunter fallen beispielsweise das γ-L-Glutamyl-L-cysteinylglycin (Glutathion), welches u.a. auch als Anabolikum angewendet wird, oder das L-β,β'-Dithiodialanin (Dicystein), das insbesondere gegen Eiweißmangelschäden, Leberparenchymerkrankungen, Schwangerschaftstoxikosen oder Furunkulose verordnet werden kann.

Es hat sich jedoch herausgestellt, daß sich das acetylierte Cystein gegenüber der Ausgangssubstanz Cystein im Körper durch erhöhte pharmakokinetische Stabilität auszeichnet. Dies wird darauf zurückgeführt, daß der oral aufgenommene Wirkstoff nicht nur als freie Substanz, sondern darüber hinaus auch über Disulfidbrücken reversibel an Proteine gebunden oder fest in Proteine inkorporiert vorliegt. Die dabei entstandenen Disulfidbrücken zwischen dem N-Acetylcystein und den Proteinen sind zwar labil, sodaß der wieder freisetzbare Wirkstoff im Organismus seine Aktivität entfalten kann, bilden aber die Ursache dafür, daß der Wirkstoff geschützt ist. In den Arzneimitteln liegen diese Wirkstoffe jedoch nicht in ihrer physiologischen Schutzform vor, sodaß jene schon während der Lagerung des sie enthaltenden Arzneimittels in Form von Granulaten, Komprimaten, Lösungen odgl. erheblichen nachteiligen Einflüssen beispielsweise durch Feuchtigkeit, Wärme, Sauerstoff oder atmosphärischen oxidativen Schadstoffen im Zusammenhang mit Ozon, z.B. Singulettsauerstoff, Sauerstoffradikalen Stickoxiden und anderen Umweltnoxen ausgesetzt sind.

Das vorstehend erwähnte Dicystein weist zwar bereits die genannte Disulfidbrücke auf, diese ist aber ebenfalls gegenüber den vorstehend aufgeführten Umwelteinflüssen und schädlichen Lagerungseinwirkungen empfindlich. Dies gilt insbesondere gegenüber Licht oder Beleuchtung, Wärme oder Säuren, wie dies bei der beliebten Einnahme derartiger Präparate in Tees oder als Brausezubereitungen zur Behandlung der vorstehend genannten Indikationen in äußerst nachteiliger Weise der Fall ist. Dies trifft insbesondere dann zu, wenn entsprechend der Empfehlung des Beipackzettels im Arzneimittel das im Handel erhältliche Brausepulver oder -granulat bzw. -tabletten oder Granulat, Tabs oder andere Instantzubereitungen mit einem Gehalt an den jeweiligen Wirkstoffen zur Einnahme in Wasser, heißem Tee oder in Fruchtsäften aufgelöst werden sollen.

So wurde gemäß EP-A-0 349 797 festgestellt, daß N-Acetylcystein-Zubereitungen bei der Einnahme mit Tee relativ instabil sind, sodaß bei längerem Stehenlassen der Zubereitung ein beträchtlicher Wirkstoffabbau zu verzeichnen ist. Dieses Problem wird gemäß dieser Publikation durch die Zugabe von Ascorbinsäure bzw Ascorbat gelöst, wobei die Mengenanteile an Ascorbinsäure zum Wirkstoff in bevorzugter Weise 5 bis 100 Teile und noch bevorzugter 10 bis 50 Teile Ascorbinsäure zu 100 Teilen betragen. Dabei werden gemäß der Vergleichskurven Einzelgaben von 25, 50 und 100 mg Ascorbinsäure erreicht.

Es hat sich in der Praxis herausgestellt, daß die in der obigen Druckschrift EP-A-0 349 797 vorgeschlagenen Stabilisationsmaßnahmen aus den folgenden Gründen in vielen Fällen unzureichend sind: Mit steigenden Mengen an Ascorbinsäure wird der pH-Wert in den sauren Bereich verschoben, sodaß bestimmte oxidative Einflüße vermehrt auftreten; darüber hinaus kann der saure Geschmack der Zubereitung in unangenehmer Weise für eine Reihe von Patienten inakzeptabel sein, insbesondere bei einer Einnahme in Früchtetee, Fruchtsäften usw, wobei in gegenläufiger Weise die Stabilität von Verbindungen, wie beispielsweise des obenstehend erwähnten Dicysteins besonders im sauren Milieu nachteilig beeinflußt wird; des weiteren haben neuere Forschungen ergeben, daß die tägliche Vitaminaufnahme aufgrund von zum Teil unkontrollierten Anreicherungen häufig eingenommener Lebensmittel, beispielsweise bei Fruchtsäften, Fertigkost, Früchtetees etc ein unter physiologischem Aspekt durchaus unerwünschtes Ausmaß annehmen kann. Dies gilt besonders für die Ascorbinsäure, deren Menge in den genannten Nahrungsmitteln infolge der künstlichen Zusätze ein Vielfaches des Tagesbedarfes, sogar den erhöhten täglichen Bedarf erkrankter Personen, mit anderen Worten, die physiologisch sinnvolle Zufuhr weit überschreiten kann. Dies gilt in gleicher Weise auch für Vitamine oder deren Vorstufen, z.B. Carotinoide oder Vitamin E, die sehr verbreitet in vielfach unkontrollierter Weise, sei es ärztlich zur Vitaminmangelprophylaxe oder für therapeutische Zwecke verordnet, rezeptfrei als Vitaminpräparate oder über vitaminisierte Fruchtsäfte, Gemüsesäfte oder andere Lebensmittel eingenommen werden.

Es ist auch bekannt, daß Arzneimittel, insbesondere solche, die im Gegensatz zu Impfstoffen keine ununterbrochene Kühlkette erfordern, durch Transport und/oder Lagerung in heißen Sommermonaten selbst in an sich kühleren Ländern, wie denen nördlicherer Breitengrade, ganz abgesehen von Mittelmeerländern oder den Tropen, massiven Wirkstoffverlusten selbst nach relativ kurzen Lagerungszeiten ausgesetzt sein können. Durch ausgereifte Verpackungstechniken sind zwar ausgeklügelte Folienbeschichtungen entwickelt worden, die lange Lagerzeiten von Arzneimitteln selbst bei hohen Temperaturen und Feuchtigkeitswerten ermöglichen; diese erfordern jedoch einen aufwendigen Maschinenpark und teure Rohstoffe bei Herstellung der Folien und deren zum Teil mehrlagiger Beschichtung, aber auch beim Einsiegeln der fertigen Dosiseinheiten. Der Platzbedarf aufgrund der großen Abmessungen der Blisterpackungen ist erheblich, sodaß sich schon allein der Transport aufgrund der großen Verpackungsvolumina sehr unwirtschaftlich gestalten kann. Ein noch größeres Problem stellen dabei ökologische Schwierigkeiten dar, beispielsweise bei der aufwendigen Entsorgung von PVC oder fluorhaltigen Polymeren, sodaß der Einsatz derartiger, hinsichtlich der Lagerstabilität verbesserter Verpackungen unerwünscht geworden ist.

EP-A-0 595 766 offenbart parenterale Lösungen für Diclofenacsalze, worin Diclofenac (iii) durch Glutathion (ii) oder N-Acetylcystein (i) stabilisiert wird. Diese Lösungen führen zu einem raschen Wirkungseintritt und einer lang andauernde Wirkung von Diclofenac. Jedoch enthält dieses Dokument weder einen Hinweis zur Stabilisierung der Lösung durch ein Stabilisatorgemisch (iv), noch zu einem Stabilisatorgemisch mit synergistischer Menge an Ascorbinsäure.

Chen & Tappel: "Protection of multiple antioxidants against...peroxidation induced by CBrCl₃ in liver, lung, kidney heart and spleen", Journal of Agricultural and Food Chemistry, 1996, Bd. 44, Nr. 3, Seiten 854-858, offenbart ein Antioxidantiengemisch enthaltend Acetylstein (i) und 100 mg Ascorbinsäure-Palmitat. Es werden mögliche synergistische Wechselwirkungen zwischen Vitamin C und Vitamin E, und zwischen (i) und Vitamin E beschrieben, jedoch nicht eine reduzierte Menge von Vitamin C. Außerdem gibt es keinen Hinweis darauf, dass sich eine verbesserte Wirkung von (iii) durch die Anwesenheit von (i) erzielen lässt.

In der DE-A-35 42 309 werden reduzierte Mengen an Vitamin C in L-Cystein (i)- und Glutathion (ii)-enthaltenden Antioxidantiengemischen verwendet. Es gibt dort aber keinen Hinweis auf eine verbesserte Wirkung von (iii) durch die Anwesenheit von (i).

Die Anmelderin sah sich deshalb vor die Aufgabe gestellt, einerseits die Stabilität Cysteinylgruppen enthaltender Zubereitungen im Hinblick auf die dargelegte Problematik nicht nur zu verbessern, sondern gleichzeitig unerwünscht hohe Stabilisierungsmengen an Ascorbinsäure bzw Ascorbat herabzusetzen.

Die Aufgabe einer in vielfacher Hinsicht zu verbessernden Stabilisierung,stellt sich umso vordringlicher, als die Anmelderin herausgefunden hat, daß Cysteinylderivate zur kombinierten Verwendung bei der Verabreichung von nichtsteroidalen Entzündungshemmern (NSAID) und Analgetika, wie beispielsweise Diclofenac, geeignet sind. Darüber hinaus hat die Anmelderin auch gefunden, daß der entzündungshemmende Effekt derartiger NSAID-Wirkstoffe durch die kombinierte Verabreichung bzw. durch entsprechende Kombinationsarzneimittel zusammen mit den Cysteinylderivaten, insbesondere Glutathion oder N-Acetylcystein in überadditiver, das heißt synergistischer Weise verstärkt wird, z.B. bei der Behandlung entzündlicher Bronchienerkrankungen. Dadurch lassen sich nicht nur die schädlichen Nebenwirkungen dieser Pharmaka in unerwarteter Weise reduzieren, sondern auch deren therapeutische Wirksamkeit, beispielsweise bei entzündlichen Erkrankungen überraschenderweise steigern. Zur Erzielung der maximal möglichen synergistischen Effekte ist es daher wünschenswert, die volle Wirkung der Cysteinylderivate, wie zum Beispiel des γ-L-Glutamyl-L-cysteinylglycins zur Entfaltung zu bringen, was nur durch geeignete weitere stabilisierende Zusätze bzw Maßnahmen bei möglichst geringen Mengen an Ascorbinsäure möglich ist.

Diese Aufgabe wird in völlig überraschender Weise dadurch gelöst, daß ein stabilisiertes Arzneimittel gemäß Anspruch 1 bereitgestellt wird, bestehend aus den Komponenten:
(i) Verbindung(en), die eine oder zwei Cysteinylgruppe(n) enthalten,
(ii) Glutathion,
(iii)einem oder mehreren nichtsteroidalen Antiphlogistika (NSAID) und/oder Analgetikum, ausgewählt aus Paracetamol, Indometacin, Acemetacin, Ibuprofen, Ketoprofen, Naproxen, Sulindac, Piroxicam, Mefenaminsäure, Phenylbutazon, Phenazon, Propyphenazon und Metamizol, sowie deren ggf. existierenden pharmakologisch aktiven Enantiomeren oder anderen optischen Isomeren, und Diclofenac,
(iv) einem Stabilisatorgemisch bestehend aus
   (a) Ascorbinsäure (Vitamin C) oder dessen Salzen oder Estern,
   (b) einem oder mehreren Tocopherolen (Vitamin E),
   (c) einem oder mehreren Carotinoiden und/oder Vitamin A, und
   (d) einem oder mehreren natürlichen oder synthetischen Flavonoiden, Bioflavonoiden, Flavanolen oder Catechinen einschließlich Anthocyanen und deren Glykosiden,
   sowie gegebenfalls
(v) für perorale, topische, parenterale oder rektale Verabreichungsformen pharmazeutisch geeignete Additive und/oder Trägerstoffe,
worin:
(α) das Mengenverhältnis der Komponenten (i), (ii), (iii) und (iv) zueinander der Bedingung genügt:

(i) : (ii) : (iii) : (iv) =
   (0, 05 - 2,0) Gew.-T. : (0, 05 - 2,0) Gew.-T. : (bis zu 2,0) Gew.-T. : (0,05 - 1,0) Gew.-T.;
(β) bezogen auf eine Dosiseinheit, die von 50 bis 1000 mg jeder einzelnen Komponente (i), (ii) oder (iii) enthält, die Menge an Stabilisatorgemisch (iv) bis zu 500 mg beträgt; und
(γ) der Anteil an Ascorbinsäure oder dessen Salzen und Estern (a) im Stabilisatorgemisch (iv) pro Dosiseinheit bis zu 50 mg beträgt.

Bei Ansprüchen 2 bis 12 handelt es sich um bevorzugte Ausführungsformen des stabilisierten Arzneimittels gemäß Anspruch 1.

In dem erfindungsgemäß stabilisiertem Arzneimittel kann der Bestandteil(b) aus einer oder mehreren Verbindungen sowie deren Derivaten natürlichen oder synthetischen Ursprungs der Reihe α-, β-, γ-, δ- und ε-Tocopherol wie auch das allrac-α-Tocopherol, Tocol, α-Tocopherolhydrochinon, α-Tocopherolchinon, deren Derivaten wie den Acetaten, Succinaten, Nicotinaten oder Poly(oxyethylen)succinaten, Ubichinonen, (Bovichinonen), (Plastochinonen), Menachinonen, ausgewählt sein.

Eine weitere erfindungsgemäße Ausgestaltung besteht darin, daß der Bestandteil(c) aus einer oder mehreren Verbindungen sowie deren Derivaten natürlichen oder synthetischen Ursprungs der Reihe Canthaxanthin, Rhodoxanthin, Capsorubin, Zeaxanthin, α-, β-, γ-, δ-, ε- und/oder ζ-Carotin, Lycopin, . Capsanthin, Cryptoxanthin, Crocetin, Lutein, Decapren-β-Carotin, Dodecapren-β-Carotin, Astaxanthin, Violaxanthin oder Bixin ausgewählt ist.

In einer bevorzugten Ausgestaltung des erfindungsgemäßen Arzneimittels ist der Bestandteil(d) aus einer oder mehreren Verbindungen sowie deren Derivaten natürlichen oder synthetischen Ursprungs der Reihe der Flavanoide bzw Bioflavanoide wie dem Chrysin, Apigenin, Fisetin, Kämpferol, Luteolin, Galangin, Gossypetin, Morin, Myricetin, Naringin, Quercetin, Robinetin, Anthocyaninen, Rutin, Hesperidin, Taxifolin, Catechin, Epicatechin, Epicatechingallat, Gallocatechin, Epigallocatechingallat, Tangeretin, Eriodictyol, Naringenin, Rutin, Troxerutin (Quercetin-rutosid), Aesculin, Aesculetin, Skimmin, Umbelliferon, entsprechenden anderen Glykosiden wie Aesculosid oder Anthocyanosiden, Rutosiden wie dem Tri-(hydroxyethyl)rutosid, Ruscogeninen, O-(β-Hydroxyethyl)-rutosiden sowie natürlichen Extrakten aus Citrusfrüchten, Myrtillum Arten, Melilotus Arten, Hypericum Arten, oder anderen Bioflavonoide liefernden Pflanzenextrakten bestehenden Gruppe ausgewählt.

Gemäß einer weiteren erfindungsgemäßen Ausgestaltung enthält das Arzneimittel ein zusätzliches Stabilisierungsmittel gemäß der Komponente(d) in Form einer oder mehrerer Verbindungen sowie deren Derivate natürlichen oder synthetischen Ursprungs, die aus der Reihe der Polyphenole wie Kaffeesäureester, Kaffeesäureamiden, Ethoxyquin, Carnosinsäure, Carhosol sowie deren Derivaten, Auszügen aus Thea Arten, Rosmarin Arten oder anderen derartige natürliche Phenolverbindungen liefernden Pflanzenextrakten ausgewählt sind.

In bevorzugter Weise können die erfindungsgemäßen stabilisierten Arzneimittel einen Gehalt an Spurenelementen, vor allem an Selen, z.B. als Natriumselenit oder -selenat aufweisen, das nicht nur einen zusätzlichen stabilisierenden Effekt auf die Arzneiform selbst ausübt, sondern auch in überadditiver Weise einen verstärkenden therapeutischen und prophylaktischen Effekt bei den vorliegend beschriebenen Indikationen z.B. bei Entzündungen oder mit toxischen Effekten freier Radikale zusammenhängenden Erkrankungen. Die Einzeldosen in mg betragen z.B. ca. 0,01, 0,02, 0,05, 0,1; die Tagesdosen in mg z.B. ca. 0,1, 0,2, 0,24, o,4, 0,5 bis maximal 0,8 oder 1,0, welch letztere Dosis 1000,0 µg entspricht.

Ferner besteht eine bevorzugte Ausgestaltung des erfindungsgemäß stabilisierten Arzneimittels darin, daß flüssige wäßrige oder Lipide enthaltende Zubereitungen Solubilisatoren einen Lösungsvermittler aus der Reihe der Phospholipide, beispielsweise Lecithinen oder Polyoxyethylenglycerolfettsäureester in Form von Monolaurat, Monostearat, Monooleat, Triricinoleat oder Trihydroxystearat (Cremophor ), Tegin -Typen, als ggf. neben organischen, pharmazeutisch verträglichen Lösungsmitteln wie Alkoholen in Form von Ethanol, Isopropanol, Butanol enthalten können. Der oder mehrere der obenstehend definierten Wirkstoffe, insbesondere das N-Acetylcystein oder der NSAID-Arzneistoff können in liposomaler, feinst verteilter oder Targetform vorliegen.

In unerwarteter Weise hat sich herausgestellt, daß die stabilisierende Wirkung der Ascorbinsäure im Vergleich zu den gemäß EP-A-0 349 797 benötigten Mengen in überadditiver Weise gesteigert ist. So ist die Menge von 10 mg, die gemäß dem in dieser Publikation dargestellten Diagramm einen stabilisierenden Effekt hat, ausreichend, die besten aus jenem Diagramm ersichtlichen Stabilitätswerte zu erreichen oder sogar zu übersteigen. Das heißt, der stabilisierende Effekt läßt sich durch die Wahl der übrigen Stabilisatorkomponenten so einstellen, daß die Ascorbinsäuremenge auf den erwünschen niedrigen Pegel beispielsweise bis herab auf 2,3 oder 5 mg pro Dosiseinheit einreguliert werden kann, wobei sich die Gesamtstabilität sogar noch steigern läßt.

Als Dosiseinheiten für die Wirkstoffe kommen im Rahmen des vorstehenden Mengenverhältnisses in bezug auf die Verbindungen (i), (ii) und (iii) jeweils beispielsweise 50 mg, 100 mg, 125 mg, 150 mg, 200 mg, 50 mg, 300 mg, 400 mg, 500 mg, 600 mg, 800 mg oder 1000 mg in Betracht. Dies gilt in typischer Weise für die oben angeführten Cysteinyl- wie auch NSAID-Verbindungen, insbesondere N-Acetylcystein und Diclofenac, aber auch für die analog strukturierten und/oder bioäquivalent wirksamen Arzneistoffe. Die jeweiligen Tagesdosen können pro Patient und einzelnem Wirkstoff bzw. den vorstehend bezeichneten Wirkstoffgruppen jeweils 300 mg, 500 mg, oder 1000 mg betragen. Dementsprechend können die Mengenanteile an dem Stabilisatorgemisch (iv) im Arzneimittel pro Dosiseinheit bzw. Einzeldosierung, wie oben für die Wirkstoffe angegeben, bis zu 500 mg betragen. Der jeweilige Anteil an Ascorbinsäure pro Dosiseinheit beträgt bis zu 50 mg.

Diese Mengenverhältnisse innerhalb des Stabilisatorgemisches wie auch der Wirkstoffe lassen sich frei variieren, sodaß die vorstehenden Anteile der Stabilisatorkomponenten und Wirkstoffdosierungen lediglich beispielhaft zu verstehen sind. Die Stabilisatormenge läßt sich je nach Wirkstoffdosis reduzieren, wie halbieren, dritteln, vierteln oder auch vervielfachen, wie verdoppeln, verdreifachen usw. Ein Stabilisatorgemisch aus 4 Komponenten kann sich pro Dosiseinheit aus 12 mg bzw. Gew.-T. (a) + 24 mg bzw. Gew.-T.(b) + 24 mg bzw. Gew.-T. (c) + 40 mg bzw. Gew.-T. (d) zusammensetzen. Die jeweiligen Einzeldosen der Komponenten des Stabilisatorgemisches können innerhalb der vorgegebenen Mengenverhältnisse, ausgedrückt in Gewichtsteilen, z.B. in Milligramm 1, 2, 3, 4, 5, 6, 10, 12, 15, 20, 25, 30, 35, 40, 50, 60, 70, 75, 80, 100 oder 120 mg sein. Bevorzugt betragen sie jedoch nicht mehr als 150 oder 200 mg für komponenten (b), (c) und (d) med nicht mehr als 150 mg für komponente (a). Dabei können diese Gewichtsteile auch jeden dazwischen befindlichen geraden oder ungeraden Wert annehmen. Das jeweils zweckmäßige Mengenverhältnis der Komponenten untereinander, beispielsweise die Ascorbinsäureverbindung zu Flavonoiden, Carotinoiden, Tocopherolen und/oder Polyphenolen und oder den entsprechenden oben erwähnten Extrakten läßt sich durch übliche Stabilisationsuntersuchungen, wie Tests unter verschärften Bedingungen, z.B. bei erhöhter Feuchtigkeit und Temperatur in relativ einfacher und bekannter Weise je nach den eingesetzten, ggf. kombinierten Wirkstoffen einstellen.

Die Wirkstoffe aus der Gruppe der Cysteinylverbindungen können getrennt von Arzneiformen bzw Dosiseinheiten mit einem Gehalt an den NSAID-Arzneistoffen zu stabilisierten Arzneimitteln verarbeitet werden, das heißt, genauer gesagt, es lassen sich in einer Arzneipackung beispielsweise orale, feste Arzneiformen, z.B. Tabletten mit einer oder mehreren Cysteinylverbindungen und Ampullen mit einem Gehalt an einer oder mehreren NSAID-Antiphlogistika getrennt nebeneinander anordnen. Mit anderen Worten, es lassen sich im Falle einer Kombination der beiden Wirkstoffgruppen das eine Cysteinylverbindung enthaltende Mittel einerseits und die ein nichtsteroidales Antiphlogistikum enthaltende Arzneiform unabhängig voneinander, z.B. etwa zum gleichen Zeitpunkt oder zeitlich verschoben, applizieren, beispielsweise das N-Acetylcystein 3 - 4 mal täglich, und das NSAID, wie Diclofenac 1 - 2 mal täglich. Das dabei einzuhaltende Therapie- und Dosisschema wird vom Arzt individuell je nach Patient, dessen Krankheitsbild und physiologischem Status festgelegt und bewegt sich im Rahmen der allgemein bekannten Verabreichungsschemata oder innerhalb der ärztlichen individuellen Dispensierung und Rezeptierung.

Im Falle der Kombination einer oder mehrerer cysteinylhaltiger Verbindungen mit den NSAID-Antiphlogistika/Analgetika kommen auch entsprechende Arzneimittel mit einem Gehalt an Stoffen aus den beiden Wirkstoffgruppen bzw. getrennt vorliegende oder zu verabreichende Arzneipackungen für die Kombinationen in Betracht, die nicht in besonderer Weise mit obigem Stabilisatorgemisch versehen sind. Die hierfür in Frage kommenden Dosierungen der Wirkstoffe entsprechen den obenstehend genannten. Im Falle der Wirkstoffkombinationen ohne Stabilisatorenzusatz ist eine verringerte Stabilität und bei verschiedenen untenstehend genannten medizinischen Indikationen, insbesondere solchen im Zusammenhang zur Unterdrükkung der Bildung freier Radikale oder deren Unschädlichmachen bzw. Entgiften/Detoxikation eine verminderte Aktivität in Kauf zu nehmen. Für die Wirkstoffkombinationen der Verbindungsgruppen (i) plus (ii) plus (iii) gelten die vorstehend genannten Dosierungen, Dosiseinheiten und Mengenverhältnisse in gleicher Weise.

Die so stabilisierten Arzneimittel können in Form von ggf. überzogenen, magensaftresistenten und/oder retardierten Kapseln, Komprimaten, Sublingual- oder Kautabletten, Kaugummi, Pastillen, Brausezubereitungen, Tabs, Granulaten, Pellets, Mikrokapseln, Trockensaft, Pulver, Injektions- und Infusionspräparaten, Suppositorien, Rektal- oder Vaginalkapseln, Tropfen, Suspensionen, Lösungen, Dosieraerosolen, Verneblern, Zerstäubern, Sprays, Liposomenzubereitungen, transdermalen Zubereitungen, Pflastern, Pasten, Emulsionen, Cremes oder Gelen, Otologika, Ophthalmologika, Ovula oder Instillationslösungen vorliegen.

Die erfindungsgemäßen Arzneiformen können insbesondere in Form von Granulat, Trockensaft, Pellets oder Liposomenzubereitung, einer Brausetablette, Brausegranulat oder Tabs vorliegen, wobei der Wirkstoff oder die Wirkstoffe (i) als Verbindung, die eine Cysteinylgruppe enthält, in Einzeldosierungen von 10mg, 20mg, 25mg, 50mg, 100 mg oder 200 mg oder der jeweils 2-, 3-, 4- oder 5- fachen Menge davon, insbesondere als N-Acetylcystein zusammen mit dem Stabilisatorgemisch aus den Komponenten (a), (b), (c) und (d) aus der Reihe Ascorbinsäureverbindung, Vitamin A (Carotinoide), Vitamin E (Tocopherole) und/oder einem oder mehreren Flavonoiden wie Rutin, Aesculin, Catechin, pflanzlichen Polyphenolen wie Kaffeesäure und/oder einem oder mehrerer bioflavonoidhaltige Extrakte aus Citrusfrüchten in Einzeldosierungen von jeweils 2,0mg, 2,5mg, 5,0mg, 10mg oder 20mg bzw. der jeweils 2-, 3-, 4-, 5- oder 10-fachen Menge davon, in Kombination mit Glutathion (ii) in Einzeldosierungen von 25mg 50mg, 100mg, 200mg oder 250mg bzw der jeweiligen 2-, 3- oder 4-fachen Menge davon, und in Kombination mit (iii) NSAID-Verbindungen, insbesondere mit Diclofenac jeweils in einer Menge 25mg, 50mg, 100mg oder 200mg bzw der jeweiligen 2-, 3-, 4-, 5- oder 10-fachen Menge davon zusammen mit üblichen Tablettier- und Granulierhilfsstoffen sowie im Falle von Brausezubereitungen, mit Kohlendioxid entwickelnden organischen Säuren wie Zitronen- oder Weinsäure und Carbonaten wie Natriumhydrogencarbonat, Granulierhilfsstoffen, Aromastoffen, Süßungsmitteln wie Saccharin und/oder Zuckern wie Mannit, Sorbit und erforderlichenfalls üblichen Trägerstoffen zur fertigen Arzneiform verarbeitet werden.

Bei der Verabreichung der erfindungsgemäßen, insbesondere stabilisierten Arzneimittel hat sich herausgestellt, daß diese Pharmaka eine Reihe von physiologisch bedeutsamen, wertvollen Eigenschaften und Effekten zeigen, beispielsweise eine Reduzierung von Muskelschäden durch physiologisch oxidativen Streß, Hintanhalten von diabetischen Gefäßschäden, Verminderung oxidativen Stresses bei Rauchern, Verminderung von (Spät)Schäden bei Cytostatikaverabreichung, insbesondere ausgelöst durch deren Langzeitapplikation, protektive Wirksamkeit gegen Hautschäden durch übermäßige UV-Strahlung oder andere schädliche Bestrahlungen oder Witterungseinflüße, vornehmlich bedingt durch oxidative Einwirkungen von Luftverunreinigungen, beispielsweise durch Ozon, Stickoxide, Sauerstoffradikale, Singulettsauerstoff und andere aggressive Radikale.

Als weiterer günstiger Effekt des erfindungsgemäßen, eine Cysteinylverbindung enthaltenden, insbesondere stabilisierten Arzneimittels mit der damit verbundenen Erhöhung des Antioxidantienstatus im Organismus ist eine günstige Reduzierung der Entzündungsmediatorensynthese und Bildung von Thromboxan sowie des die Zellproliferierung fördernden Fakors PDGF (platelet-derived growth factor) im Zusammenhang mit der Zufuhr von ω-3-polyungesättigten Fettsäuren (ω-3-PUFA) zu verzeichnen, sei es, daß die Aufnahme mit der Nahrung, sei es in Form von arzneilichen Ergänzungsmitteln erfolgt. Dies gilt auch für eine in diesem Falle eintretende Senkung erhöhter Fibrinogenkonzentrationen, sodaß die Wirkung der NSAID in unerwarteter Weise durch die erfindungsgemäße Kombination insbesondere mit einem Gehalt an Glutathion oder dessen physiologischem Vorläufer bzw. dessen Estern als Cysteinylverbindungen begünstigt wird.

Die letztgenannten Cysteinderivate und damit vergleichbare Cysteinylverbindungen spielen eine Rolle bei den antioxidativen Schutzmechanismen bei durch Lungen- und Bronchialerkrankungen, übermäßigem Tabakrauchen, Ozonbelastungen und toxischen, in der Luft vorhandenen Radikalen verursachten Entzündungsprozessen und Noxen, die ebenfalls mit verstärkter Entzündungsmediator-, Entzündungszellen- und freie Radikalproduktion verbunden sind. Es wurde erfindungsgemäß festgestellt, daß bereits die alleinige Verabreichung vorgenannter Cysteinylverbindungen zusammen mit dem erfindungsgemäßen Antioxidantiengemisch ohne weitere Wirkstoffe, d.h. auch ohne NSAID, entzündungshemmende Effekte bewirkt und ferner, daß die durch Entzündungen verursachten physiologischen Schäden in jeweils überadditiver Weise verringert werden.

Insbesondere eignet sich das erfindungsgemäße stabilisierte Arzneimittel zur Behandlung von Otitis, Sinusitis, Laryngitis, Mukoviszidose und Schmerzzuständen.

Dabei sind diese vorteilhaften Wirkungen insbesondere an verschiedenen Organen, wie Lunge, Niere, Leber oder Herz zu verzeichnen, welche Organe durch entsprechend nachteilige Umwelteinflüße, wie Luft-, Wasser- und Nahrungsmittelschadstoffen besonders starken Belastungen unterworfen sind, die der Organismus aus eigener Kraft oft nicht mehr zu bewältigen imstande ist, sodaß aufgrund der Einwirkung von Umweltnoxen zahlreiche, z.T. irreparable Organschäden und chronische Erkrankungen an der Tagesordnung sind. Dies gilt umso mehr, als der in zahlreichen Lebensmitteln, z.B. Gemüse oder Früchten natürlich vorkommende Gehalt an beispielsweise Glutathion durch Lagerung oder Bearbeitung stark vermindert sein kann. Die erfindungsgemäßen cysteinylhaltigen Arzneimittel eignen sich deshalb nicht nur zur überadditiven Substitution cysteinylhaltiger Substanzen, sondern auch ganz hervorragend zur Prophylaxe und Therapie von streßbedingten, bevorzugt von entzündlichen Erkrankungen der vorstehend genannten Organe wie Pneumonie, Hepatitis, Nephritis, Arteriosklerose, Venenerkrankungen, Arteriitis unterschiedlicher Genese oder auch Immun- bzw. Autoimmunerkrankungen, Transplantatabstoßungen, insbesondere entzündlicher Art, diabetischer Spätfolgen an Gefäßen, Niere und Retina etc, Strahlenschäden, Perfusions- oder Reperfusionsschäden, besonders auch zum Kompensieren von durch Arzneimittel bedingten Folgeschäden, z.B. nach Antibiotika-, Analgetika- oder Cytostatikaverabreichung usw. Die erfindungsgemäßen stabilisierten cysteinylhaltigen Arzneimittel eignen sich auch zum Entgiften nach Toxizitätseinwirkungen der verschiedensten Noxen, beispielsweise u.a. von zytotoxischen Lipidperoxidationsprodukten. Die vorliegenden erfindungsgemäß stabilisierten Zubereitungen stellen deshalb auch ein ausgezeichnetes Puffersystem für den Redoxzustand der Zelle aufgrund der vorhandenen SH- und Disulfidgruppen dar, sodaß die vorstehend beschriebenen Effekte besonders stark wirksam werden. Bevorzugt lassen sich die erfindungsgemäßen, insbesondere stabilisierten, Cysteinylverbindungen enthaltenden Arzneimittel im Zusammenhang mit einer Inhibierung oder Prophylaxe der Apoptose in vorteilhafter Weise einsetzen, zum Beispiel im Gefolge einer arzneilichen Normalisierung der Koronardurchblutung, wobei das an einen Infarktbezirk angrenzendes Gewebe infolge der Durchblutungssteigerung vermehrt mit schädlichen freien Radikalen überschwemmt wird. Dieser toxische Effekt läßt sich durch die erfindungsgemäßen Arzneimittel in günstiger Weise therapeutisch und prophylaktisch beeinflussen. Die Zell- bzw. Gewebsmechanismen der Apoptosevorgänge sind beispielsweise in "Apoptosis and Programmed Cell Death in Immunity", J.J.Cohen, R.C.Duke, V.A.Fadok und K.S.Sellins, Annual Review of Immunology, Bd.10, (1992), Seiten 267-293 oder "Apoptosis: A Basic Biological Phenomenon with Wide-Ranging Implications, British Journal of Cancer, Bd.26, (1972), Seiten 239-257 eingehend beschieben.

Die Wirkung des erfindungsgemäß mit der Antioxidantienmischung stabilisierten Arzneimittels läßt sich weiter durch die Herstellung von Liposomenzubereitungen steigern, wobei feinste Teilchen der Cysteinylverbindungen und/oder NSAID-Wirkstoffe in üblicher Weise mit einer Membran überzogen werden.

Es konnte auch festgestellt werden, daß sich die erfindungsgemäßen cysteinylhaltigen Pharmaka in Form von flüssigen Zubereitungen, z.B. Lösungen mit einem Gehalt an der obenstehend definierten Antioxidantienmischung zur verlängerten Konservierung oder der Perfusion von Organen zur autogenen oder allogenen Transplantation, beispielsweise von Herz, Leber, Niere, Lunge, Gefäßen, wie Venen, Pankreas oder Inselzellen geeignet sind.

Eine weitere medizinische Indikation des erfindungsgemäßen, insbesonderestabilisierten Arzneimittels aufgrund seiner detoxifizierenden Wirkung auf Kanzerogene ist zur Prophylaxe des Bronchialkarzinoms und anderer Karzinome applizierbar. Bei der Therapie jener speziellen neoplastischen Neubildung kann, wie obenstehend ausgeführt, das erfindungsgemäße Arzneimittel auch in Kombination mit einem Cytostatikum appliziert werden. Somit ergibt sich nicht nur eine Unterdrückung bei der Entstehung oder der Wirkung bereits vorhanderer carcinogener Substanzen, sondern gleichzeitig auch eine vorteilhafte entgiftende Wirkung der teilweise mit starken Nebenwirkungen behafteten Cancerostatika, Immunsuppressiva und Cytostatika.

Aufgrund der besonderen Eigenschaften des erfindungsgemäßen Arzneimittels übt dieses auch ohne zusätzlichen Gehalt an NSAID eine prophylaktische und therapeutische Wirkung bei Entzündungserkrankungen wie rheumatischen Erkrankungen, z.B. Arthritiden oder Venenerkrankungen, hämorrhoidaler Symptomatik, Thrombopathien oder Thrombosen aus, sodaß eine Anwendung bei gegenüber üblichen Antirheumatika bei sensiblen Patienten eine unerwartete Verkürzung des Erkrankungsverlaufes zu verzeichnen ist. Dies ist, wie oben ausgeführt, eine weitere günstige, vorteilhafte Eigenschaft des vorliegenden Arzneimittels insbesondere mit einem Gehalt an dem Stabilisatorgemisch.

Bezüglich des Standes der Technik zu liposomalen Zubereitungen, beispielsweise von N-Acetylcystein-Liposomen, sei auf die US-A-4 895 719 (=EP-A-0 223 831) und die Publikation WO-A-9 116 882 hingewiesen, deren Herstellungsverfahren auch zur Herstellung der erfindungsgemäßen stabilisierten Arzneimittel dienen kömmen.

### Therapeutische Darreichungsformen

Die Herstellung von Arzneimitteln mit einem Gehalt an den obenstehend genannten Verbindungen erfolgt in üblicher Weise anhand geläufiger pharmazeutisch-technischer Verfahren. Dazu werden die Wirkstoffe als solche oder in Form ihrer Salze zusammen mit geeigneten, pharmazeutisch annehmbaren Hilfs- und Trägerstoffen zu den für die verschiedenen Indikationen und Applikationsorten geeigneten Arzneiformen verarbeitet. Dabei können die Arzneimittel in der Weise hergestellt werden, daß die jeweils erwünschte Freisetzungsrate, z.B. eine rasche Anflutung und/oder ein Retard- bzw. Depoteffekt erzielt werden. Dabei kann die Steuerung der kontrollierten Freisetzung über längere oder kürzere Zeiträume und an verschiedenen Stellen des Intestinaltraktes, wie Magen und verschiedenen Darmabschnitten durch die Mischung unterschiedlich freisetzender Pelletsorten, polymere Überzüge, pH-abhängige Substanzen, Kern/Manteltabletten, Mehrschichttabletten, polymere und/oder lipide Matrix- bzw. Gerüstsubstanzen oder kombinierte Retardmaßnahmen erfolgen.

Bevorzugt ist im Falle von Wirkstoffkombinationen die getrennte Verarbeitung der Wirkstoffe (i) und (ii) mit dem Stabilisator einerseits und eines oder mehrerer kombinierter Wirkstoffe (iii) andererseits. Das heißt, die Stabilität der Wirkstoffe (i) und (ii) läßt sich durch die getrennte Herstellung beispielsweise eines Granulats, von Pellets oder überzogenen Pulvern, Liposomen usw., enthaltend (i) und (ii), den Cysteinylverbindungen einerseits und eines zweiten Granulats, enthaltend (iii), NSAID andererseits, weiter steigern.

Neben peroral verabreichbaren Arzneiformen können auch Injektabilia verabreicht werden. Diese können entweder in Form von Ampullen oder auch als sog. gebrauchsfertige Injektabilia, z.B. als Fertigspritzen oder Einmalspritzen, daneben auch in Durchstechflaschen zur mehrmaligen Entnahme hergerichtet sein. Die Verabreichung der Injektabilia oder von Infusionslösungen kann in Form der subkutanen (s.c.), intramuskulären (i.m.), intravenösen (i.v.) oder intrakutanen (i.c.) Applikation erfolgen. Die jeweils zweckmäßigen Injektionsformen können insbesondere als Lösungen, Kristallsuspensionen, nanopartikuläre oder kolloiddisperse Systeme, wie z.B. Hydrosole, hergestellt werden.

Die injizierbaren Zubereitungen können auch als Konzentrate hergestellt werden, welche mit wäßrigen isotonischen Verdünnungsmitteln auf die gewünschte Wirkstoffdosierung eingestellt werden können. Weiterhin können sie auch als Pulver, wie z.B. Lyophilisate, hergestellt werden, die dann vorzugsweise unmittelbar vor der Applikation mit geeigneten Verdünnungsmitteln aufgelöst oder dispergiert werden. Die Infusionen lassen sich ebenfalls in Form von isotonischen Lösungen, Fettemulsionen, Liposomenzubereitungen, Mikroemulsionen, Flüssigkeiten auf Basis von Mischmizellen, z.B. auf Basis von Phospholipiden, zubereiten. Wie Injektabilia können auch Infusionszubereitungen in Form von Konzentraten zum Verdünnen zubereitet werden. Die injizierbaren Zubereitungen können auch in Form von Dauerinfusionen sowohl in der stationären als auch in der ambulanten Therapie, z.B. in Form von Minipumpen, appliziert werden.

Den parenteralen Arzneiformen können beispielsweise Albumin, Plasmaexpander, oberflächenaktive Stoffe, organische Lösungsmittel, pH-beeinflussende Stoffe, komplexbildende Stoffe oder polymere Stoffe, insbesondere als Substanzen zur Beeinflussung der Adsorption des Wirkstoffes an Protein oder Polymeren oder auch mit dem Ziel hinzugefügt werden, die Adsorption des Wirkstoffes an Materialien, wie Injektionsbestecke oder Verpackungsmittel, beispielsweise Kunststoff oder Glas, zu verringern.

Die Wirkstoffe können in den Parenteralia an Nanopartikel gebunden sein, beispielsweise an feinstverteilte Partikel auf Basis von Poly(meth)acrylaten, Polylactaten, Polyglycolaten, Polyaminsäuren oder Polyetherurethanen. Die parenteralen Zubereitungen können auch als Depotpräparate modifiziert sein, z.B. aufbauend auf dem multiple unit Prinzip, wenn die Wirkstoffe in feinst verteilter bzw. dispergierter, suspendierter Form oder als Kristallsuspension eingearbeitet sind oder aufbauend auf dem single unit Prinzip, wenn der Wirkstoff eingeschlossen ist in eine Arzneiform, z.B. eine Tablette oder ein Stäbchen, das anschließend implantiert wird. Häufig bestehen diese Implantate oder Depotarzneimittel bei single unit und multiple unit Arzneiformen aus sogenannten bioabbaubaren Polymeren, wie z.B. Polyester der Milch- und Glykolsäure, Polyetherurethanen, Polyaminosäuren, Poly(meth)acrylaten oder Polysacchariden.

Als Hilfs- und Trägerstoffe bei der Herstellung von Parenteralia kommen Aqua sterilisata, den pH-Wert beeinflussende Substanzen, wie z.B. organische und anorganische Säuren und Basen sowie deren Salze, Puffersubstanzen zur Einstellung des pH-Wertes, Isotonisierungsmittel, wie z.B. Natriumchlorid, Natriumhydrogencarbonat, Glucose und Fructose, Tenside bzw. oberflächenaktive Substanzen und Emulgatoren, wie z.B. Partialfettsäureester des Polyoxyethylensorbitans (Tween® ) oder z.B. Fettsäureester des Polyoxyethylens (cremophor® ), fette Öle, wie z.B. Erdnußöl, Sojabohnenöl und Rizinusöl, synthetische Fettsäureester, wie z.B. Ethyloleat, Isopropylmyristat und Neutralöl (Miglyol® ), sowie polymere Hilfsstoffe wie z.B. Gelatine, Dextran, Polyvinylpyrrolidon, von die Löslichkeit erhöhenden Zusätzen organischer Lösungsmittel wie z.B.

Propylenglycol, Ethanol, N,N-Dimethylacetamid, Propylenglycol oder komplexbildender Stoffe, wie z.B. Citraten und Harnstoff, Konservierungsmittel, wie z.B. Benzoesäurehydroxypropyl- und
-methylester, Benzylalkohol, weitere Antioxidantien, wie z.B. Natriumsulfit und Komplexbildner als Stabilisatoren, wie z.B. EDTA, in Betracht.

Bei Suspensionen erfolgt ein Zusatz von Verdickungsmitteln zum Verhindern des Absetzens der Wirkstoffe von Tensiden und Peptisatoren, um die Aufschüttelbarkeit des Sediments zu sichern, oder von Komplexbildnern wie EDTA. Es lassen sich auch mit verschiedenen Polymeren Wirkstoffkomplexe erzielen, beispielsweise mit Polyethylenglykolen, Polystyrol, Carboxymethylcellulose, Pluronics® oder Polyethylenglykolsorbitanfettsäureestern. Der Wirkstoff läßt sich auch in Form von Einschlußverbindungen, z.B. mit Cyclodextrinen, in flüssige Zubereitungen inkorporieren. Als weitere Hilfsstoffe kommen auch Dispergiermittel in Betracht. Zur Herstellung von Lyophilisaten werden Gerüstbildner, wie z.B. Mannit, Dextran, Saccharose, Humanalbumin, Lactose, PVP oder Gelatinesorten verwendet.

Soweit die Wirkstoffe nicht in Form der Base in die flüssigen Arzneizubereitungen eingearbeitet werden, lassen sie sich in Form ihrer Säureadditionssalze, Hydrate oder Solvate in den Parenteralia einsetzen.

Eine weitere systemische Applikationsform von Bedeutung ist die perorale Verabreichung als Tabletten, Hart- oder Weichgelatinekapseln, Dragees, Pulver, Pellets, Mikrokapseln, Oblongkomprimate, Granulate, Kautabletten, Lutschpastillen, Kaugummi, Sublingualtabletten oder Sachets. Diese festen peroral verabreichbaren Formen lassen sich auch als Retard- bzw. Depotsysteme herrichten. Dazu zählen Arzneimittel mit einem Gehalt an einem oder mehreren mikronisierten Wirkstoffen, Diffusions- und Erosionsformen auf Matrixbasis, z.B. unter Verwendung von Fetten, wachsartigen und/oder polymeren Stoffen, oder sog. Reservoirsysteme. Als Retardiermittel bzw. Mittel zur gesteuerten Freisetzung kommen film- oder matrixbildende Substanzen, wie z.B. Ethylcellulose, Hydroxypropylmethylcellulose, Poly(meth)acrylatd.erivate (z.B. Eudragit® ), Hydroxypropylmethylcellulosephthalat sowohl in organischen Lösungen als auch in Form wäßriger Dispersionen in Frage. In diesem Zusammenhang sind auch sogenannte bioadhäsive Präparate zu nennen, bei denen die erhöhte Verweilzeit im Körper durch intensiven Kontakt mit den Körperschleimhäuten erreicht wird. Ein Beispiel eines bioadhäsiven Polymers ist z.B. die Gruppe der Carbomere® .

Zur sublingualen Applikation sind insbesondere Komprimate, wie z.B. nicht-zerfallende Tabletten in Oblongform geeigneter Größe mit langsamer Wirkstofffreigabe geeignet. Zum Zwecke einer gezielten Freisetzung der Wirkstoffe in den verschiedenen Abschnitten des Gastrointestinaltraktes lassen sich Mischungen aus an den verschiedenen Orten freisetzenden Pellets, z.B. Gemische aus magensaftlöslichen und dünndarmlöslichen bzw. magensaftresistenten und dickdarmlöslichen einsetzen. Dasselbe Ziel der Freisetzung an verschiedenen Abschnitten des Gastrointestinaltraktes läßt sich auch durch entsprechend hergestellte Manteltabletten mit Kern konzipieren, wobei der Mantel den Wirkstoff im Magensaft schnell freisetzt und der Kern den Wirkstoff im Dünndarmmilieu allmählich freigibt. Das Ziel einer gesteuerten Freisetzung an verschiedenen Abschnitten des Gastrointestinaltrakts läßt sich auch durch Mehrschichttabletten erreichen. Die Pelletgemische mit unterschiedlich freigesetztem Wirkstoff lassen sich in Hartgelatinekapseln abfüllen.

Als weitere Hilfsstoffe zur Herstellung von Komprimaten, wie z.B. Tabletten oder Hart- und Weichgelatinekapseln sowie Dragees und Granulaten werden beispielsweise Gegenklebe- und Schmier- und Trennmittel, Dispergiermittel, wie z.B. flammendisperses Siliziumdioxid, Sprengmittel, wie z.B. verschiedene Stärkearten, PVP, Celluloseester als Granulier- oder Retardiermittel, wie z.B. wachsartige und/oder polymere Stoffe auf Eudragit® -, Cellulose- oder Cremophor® -Basis eingesetzt.

Antioxidantia, Süßungsmittel, wie z.B. Saccharose, Xylit oder Mannit, Geschmackskorrigenzien, Aromastoffe, Konservierungsmittel, Farbstoffe, Puffersubstanzen, Direkttablettiermittel, wie z.B. mikrokristalline Cellulose, Stärke und Stärkehydrolysate (z.B. Celutab® ), Milchzucker, Polyethylenglykole, Polyvinylpyrrolidon und Dicalciumphosphat, Gleitmittel, Füllstoffe, wie z.B. Lactose oder Stärke, Bindemittel in Form von Lactose, Stärkearten, wie z.B. Weizen- oder Mais- bzw. Reisstärke, Cellulosederivate, wie z.B. Methylcellulose, Hydroxypropylcellulose oder Kieselerde, Talkum, Stearate, wie z.B. Magnesiumstearat, Aluminiumstearat, Calciumstearat, Talk, silikonisierter Talk, Stearinsäure, Cetylalkohol, hydrierte Fette verwendet.

In diesem Zusammenhang wären auch orale therapeutische Systeme, insbesondere aufbauend auf osmotischen Prinzipien, wie z.B. GIT (gastrointestinales therapeutisches System) oder OROS (orales osmotisches System) zu erwähnen.

Zu den peroral verabreichbaren Komprimaten zählen vor allem Brausetabletten, Brausegranulate und Tabs, welche beide rasch in Wasser lösliche oder suspendierbare und sofort trinkbare Instantarzneiformen darstellen. Als Tabletten- und Granulierhilfstoffe werden zur Herstellung der genannten Brauseformen unter anderem Systeme zur Entwicklung von gasförmigem, in wäßrigem Milieu in situ gebildetem CO₂, wie organische Säuren, z.B. Zitronensäure und Carbonate eingesetzt.

Zu den peroral verabreichbaren Formen zählen auch Lösungen, z.B. Tropfen, Säfte und Suspensionen, die nach den obenstehend angegebenen Verfahren hergestellt werden und zur Erhöhung der Stabilität noch Konservierungsmittel und gegebenenfalls aus Gründen der erleichterten Einnahme noch Aromastoffe und zur besseren Unterscheidbarkeit Farbstoffe sowie Antioxidantia und/oder zusätzlich zu den in dem Stabilisatorgemisch enthaltenen noch weitere Vitamine und Süßstoffe, wie Zucker oder künstliche Süßungsmittel enthalten können. Dies gilt auch für Trockensäfte, die vor der Einnahme mit Wasser zubereitet werden. Zur Herstellung flüssig einzunehmender Formen wären auch Ionenaustauscherharze in Verbindung mit einem oder mehreren Wirkstoffen zu erwähnen.

Eine spezielle Abgabeform besteht in der Herrichtung von sog. Schwimmarzneiformen, beispielsweise auf Basis von Tabletten oder Pellets, die nach Kontakt mit Körperflüssigkeiten Gase entwickeln und deshalb an der Oberfläche der Magenflüssigkeit schwimmen. Weiterhin können auch sogenannte elektronisch gesteuerte Abgabesysteme formuliert werden, bei denen die Wirkstoffabgabe über externe elektronische Impulse gezielt auf die individuellen Bedürfnisse eingestellt werden können.

Eine weitere Gruppe systemisch verabreichbarer und gegebenenfalls auch topisch wirksamer Arzneiformen stellen rektal applizierbare Arzneimittel dar. Dazu zählen die Suppositorien und Klistierzubereitungen. Die Klistierzubereitungen können auf Basis von Tabletten mit wäßrigen Lösungsmitteln zum Herstellen dieser Verabreichungsform hergerichtet werden. Auf der Grundlage von Gelatine oder anderen Trägerstoffen lassen sich auch Rektalkapseln bereitstellen.

Als Suppositoriengrundlagen kommen Hartfette, wie z.B. Witepsol® , Massa Estarinum® , Novata® , Kokosfett, Glycerin-Gelatine-Massen, Glycerin-Seifen-Gele und Polyethylenglykole in Betracht.

Als topisch, lokal oder regional verabreichbare Arzneimittel kommen als spezielle Zubereitungen die folgenden in Betracht: vaginal oder genital applizierbare Emulsionen, Cremes, Schaumtabletten, Depotimplantate, Ovula oder transurethral verabreichbare Instillationslösungen. Für die ophthalmologischen Applikationen eignen sich streng sterile Augensalben, - lösungen bzw. -tropfen oder -cremes und -emulsionen.

In gleicher Weise können für die Applikation am Ohr oder in der Nase entsprechende otologische Tropfen, Salben oder Cremes vorgesehen werden. Für die beiden vorstehend genannten Applikationen ist auch die Verabreichung von halbfesten Zubereitungen, wie z.B. Gelen auf Basis von Carbopol® -Typen oder anderen Polymerverbindungen, wie z.B. Polyvinylpyrrolidon und Cellulosederivaten möglich.

Zur herkömmlichen Applikation auf der Haut oder auch der Schleimhaut lassen sich übliche Emulsionen, Gele, Salben, Cremes oder mischphasige bzw. amphiphile Emulsionssysteme (Öl/Wasser-Wasser/Öl-Mischphase) sowie Liposomen und Transfersomen nennen. Als Hilfs- bzw. Trägerstoffe eignet sich beispielsweise Natriumalginat als Gelbildner zur Herstellung einer geeigneten Grundlage oder Cellulosederivate, wie z.B. Guar- oder Xanthangummi, anorganische Gelbildner, wie z.B. Aluminiumhydroxide oder Bentonite (sog. thixotrope Gelbilder), Polyacrylsäurederivate, wie z.B. Carbopol® , Polyvinylpyrrolidon, mikrokristalline Cellulose oder Carboxymethylcellulose. Weiterhin kommen amphiphile nieder- und höhermolekulare Verbindungen sowie Phospholipide in Betracht. Die Gele können entweder als Hydrogele auf Wasserbasis oder als hydrophobe Organogele, beispielsweise auf Basis von Gemischen nieder- und hochmolekularer Paraffinkohlenwasserstoffe und Vaseline vorliegen.

Derartige pharmazeutische bzw. dermatologische Zubereitungen eignen sich nicht nur hervorragend als Arzneimittel bei den vorstehend genannten Indikationen, sondern auch als ausgezeichnete dermale oder muköse Schutzfilme oder schützende Abdeckungen gegenüber aggressiven Umwelteinflüßen.

Für die Herstellung dieser Mittel lassen sich als Emulgatoren anionische, kationische oder neutrale Tenside, beispielsweise Alkaliseifen, Metallseifen, Aminseifen, sulfurierte und sulfonierte Verbindungen, Invertseifen, hohe Fettalkohole, Partialfettsäureester des Sorbitans und Polyoxyethylensorbitans, z.B. Lanette-Typen, Wollwachs, Lanolin oder andere synthetische Produkte zur Herstellung der Öl/Wasser- und/oder Wasser/Öl-Emulsionen einsetzen.

Hydrophile Organogele können beispielsweise auf Basis hochmolekularer Polyethylenglykole zubereitet werden. Diese gelartigen Formen sind abwaschbar. Als Lipide in Form fett- und/oder öl- und/oder wachsartiger Komponenten zur Herstellung der Salben, Cremes oder Emulsionen werden Vaseline, natürliche oder synthetische Wachse, Fettsäuren, Fettalkohole, Fettsäureester, z.B. als Mono-di- oder -triglyceride, Paraffinöl oder vegetabilische Öle, gehärtetes Rizinusöl oder Kokosöl, Schweinefett, synthetische Fette, z.B. auf Capryl-, Caprin-, Laurin- und Stearinsäurebasis wie z.B. Softisan® oder Trigylceridgemischen wie Miglyol® eingesetzt.

Zur Einstellung des pH-Wertes können osmotisch wirksame anorganische oder organische Säuren sowie Laugen , z.B. Salzsäure, Citronensäure, Natronlauge, Kalilauge, Natriumhydrogencarbonat, ferner Puffersysteme, wie z.B. Citrat, Phosphat, Tris-Puffer oder Triethanolamin verwendet werden.

Zur Erhöhung der Stabilität können noch Konservierungsmittel, z.B. wie Methyl oder Propylbenzoat (Parabene) oder Sorbinsäure hinzugesetzt werden.

Als weitere topisch applizierbare Formen lassen sich Pasten, Puder oder Lösungen erwähnen. Die Pasten enthalten als konsistenzgebende Grundlagen oft lipophile und hydrophile Hilfsstoffe mit sehr hohem Feststoffanteil.

Die Puder oder topisch applizierbare Pulver können zur Erhöhung der Dispersität sowie des Fließ- und Gleitvermögens sowie zur Verhinderung von Agglomeraten, z.B. Stärkearten, wie Weizen- oder Reisstärke, flammendisperses Siliziumdioxid oder Kieselerden, die auch als Verdünnungsmittel dienen, enthalten.

Als nasale Applikationsformen dienen Nasentropfen oder Nasensprays. In diesem Zusammenhang können auch Vernebler, Dosieraerosole oder Nasencreme oder -salbe zur Verwendung gelangen.

Nasenspray oder Trockenpulverzubereitungen sowie Dosieraerosole eignen sich darüber hinaus auch zur systemischen Verabreichung der Wirkstoffe.

Diese Druck- bzw. Dosieraerosole und Trockenpulverzubereitungen können inhaliert bzw. insuffliert werden. Derartige Verabreichungsformen haben auch für die direkte, regionale Applikation in der Lunge oder Bronchien und Kehlkopf eine gewisse Bedeutung erlangt. Dabei können die Trockenpulverzusammensetzungen beispielsweise als Wirkstoff-Softpellets, als Wirkstoff-Pulvermischung mit geeigneten Trägerstoffen, wie z.B. Lactose und/oder Glukose formuliert werden. Für die Inhalation oder Insufflation eignen sich übliche Applikatoren, die sich zur Behandlung des Nasen-, Mund- und/oder Rachenraums eignen. Die Wirkstoffe lassen sich auch mittels eines Ultraschallvernebelungsgerätes applizieren. Als Treibgase für Aerosol-Sprühformulierungen bzw. Dosieraerosole eignen sich z.B. Tetrafluorethan oder HFC 134a und/oder Heptafluorpropan oder HFC 227, wobei nichtfluorierte Kohlenwasserstoffe oder andere bei Normaldruck und Raumtemperatur gasförmige Treibmittel, wie z.B. Propan, Butan oder Dimethylether bevorzugt sein können. Anstelle der Dosieraerosole lassen sich auch treibgasfreie, manuelle Pumpsysteme verwenden.

Zweckmäßigerweise können die Treibgasaerosole auch oberflächenaktive Hilfsstoffe enthalten, wie z.B. Isopropylmyristat, Polyoxyethylensorbitanfettsäureester, Sorbitantrioleat, Lecithine oder Sojalecithin.

Für die regionale Applikation in situ sind z.B.Lösungen zur Instillation, beispielsweise zur transurethralen Verabreichung bei Blasentumoren oder Genitaltumoren, oder zur Perfusion bei Lebertumoren oder anderen Organcarcinomen geeignet.

Die jeweils geeigneten Arzneiformen lassen sich in Einklang mit den Rezepturvorschriften und Verfahrensweisen auf der Basis pharmazeutisch-physikalischer Grundlagen, wie sie beispielsweise in den folgenden Handbüchern beschrieben und in den vorliegenden Erfindungsgegenstand im Hinblick auf die Herstellung der jeweils geeigneten Arzneimittel eingeschlossen sind:
Physical Pharmacy (A.N. Martin, J. Swarbrick, A. Cammarata), 2nd Ed., Philadelphia Pennsylvania, (1970), dt. Ausgabe: Physikalische Pharmazie, (1987), 3. Auflage, Stuttgart;
R. Voigt, M. Bornschein, Lehrbuch der pharmazeutischen Technologie, Verlag Chemie, Weinheim, (1984), 5. Auflage;
P.H. List, Arzneimformenlehre, Wissenschaftliche Verlagsgesellschaft mbH, Stuttgart, (1985), 4. Auflage;
H. Sucker, P. Fuchs, P. Speiser, Pharmazeutische Technologie, Georg Thieme Verlag, Stuttgart - New York, (1991), 2. Auflage;
A.T. Florence, D. Attwood, Physicochemical Principles of Pharmacy, The Maximillan Press Ltd., Hongkong, (1981);
Hager's Handbuch der Pharmazeutischen Praxis, 5. Auflage;
L.A.Trissel, Handbook on injectable Drugs, American Society of Hospital Pharmacists, (1994), 8. Auflage;
Y.W. Chien, Transdermal Controlled Systemic Medications, Marcel Dekker Inc., New York - Basel, (1987);
K.E. Avis, L. Lachmann, H.A. Liebermann, Pharmaceutical Dosage Forms: Parenteral Medications, Volum 2, Marcel Dekker Inc., New York - Basel, (1986);
B.W. Müller, Controlled Drug Delivery, Paperback APV, Band 17, Wissenschaftliche Verlagsgesellschaft mbH, Stuttgart, (1987);
H. Asch, D. Essig, P.C. Schmidt, Technologie von Salben, Suspensionen und Emulsionen, Wissenschaftliche Verlagsgesellschaft mbH, Stuttgart, (1984);
H.A. Liebermann, L. Lachman, J.B. Schwartz, Pharmaceutical Desage forms: Tablets, Volume 1, Marcel Dekker Inc. New York, 2. Auflage (1989);
D. Chulin, M. Deleuil, Y. Pourcelot, Powder Technology and Pharmaceutical Processes, in J.C. Williams, T. Allen, Handbook of Powder Technology, Elsevier Amsterdam - London - New York - Tokyo, (1994);
J.T. Carstensen, Pharmaceutical Principles of Solid Dosage Forms, Technomic Publishing Co., Inc., Lancaster - Basel, (1993).

### HERSTELLUNGSBEISPIEL, ZUR ERLEICHTERUNG DES VERSTÄNDNIS DER ERFINDUNG

Zur Herstellung von Brausezubereitungen werden pro Dosiseinheit 50, 100, 300, 400, 500 oder 600 mg N-Acetylcystein oder die entsprechende Menge an Glutathion, 2, 5, 10, 20, 25, 30 oder 50 bis 60 mg von (a), der Ascorbinsäureverbindung, von (c), einem oder mehreren Carotinoiden, von (b), einem oder mehreren Tocopherolen, und von (d), einem oder mehreren Flavonoiden wie Rutin oder Aesculin und ggf. Polyphenolen wie Kaffeesäure(amid) oder entsprechenden Extrakten aus Citrusfrüchten oder grünem Tee in den nämlichen Dosiseinzeldosierungen in üblicher Weise mit pharmazeutisch annehmbaren Hilfsstoffen zu einem Granulat oder Pulver verarbeitet, wobei als Kohlendioxid entwickelnde Substanzen bevorzugt organische Säuren wie Zitronen- und/oder Weinsäure nebst Carbonaten, wie bevorzugt Natriumhydrogencarbonat eingesetzt und das Endgemisch erforderlichenfalls ggf. unter Zusatz von Gleit-, Gegenklebe- und Schmiermitteln sowie erforderlichenfalls weiteren üblichen Tablettierhilfsstoffen kompaktiert wird. Für die Kombination mit NSAID, insbesondere mit Di-clofenac werden pro Dosiseinheit je nach erwünschter Wirkungstärke, Wirkäquivalenz und Wirktyp bzw Pharmakokinetik eine Menge von 10, 25, 50, 100, 200, 300, 400, 500, 600 oder mehr mg bevorzugt zu einem separaten Granulat oder Pulver verarbeitet, zu dem vorstehend hergerichteten Granulat zugemischt und gewünschtenfalls in üblicher Weise zu Tabletten komprimiert. Die angegebenen Mengen an Wirkstoff(en) und Stabilisatorgemisch können erforderlichenfalls je nach Dosierung jeweils halbiert oder auch verdoppelt oder verdreifacht werden. Die so erhaltenen Arzneiformen werden vor Feuchtigkeit geschützt in üblicher Weise konfektioniert.

## Patentansprüche

1. Stabilisiertes Arzneimittel aus den Komponenten
(i) Verbindung(en), die eine oder zwei Cysteinylgruppe(n) enthalten,
(ii) Glutathion,
(iii)einem oder mehreren nichtsteroidalen Antiphlogistika (NSAID) und/oder Analgetikum, ausgewählt aus Paracetamol, Indometacin, Acemetacin, Ibuprofen, Ketoprofen, Naproxen, Sulindac, Piroxicam, Mefenaminsäure, Phenylbutazon, Phenazon, Propyphenazon und Metamizol, sowie deren ggf. existierenden pharmakologisch aktiven Enantiomeren oder anderen optischen Isomeren, und Diclofenac,
(iv) einem Stabilisatorgemisch bestehend aus
(a) Ascorbinsäure (Vitamin C) oder dessen Salzen oder Estern,
(b) einem oder mehreren Tocopherolen (Vitamin E),
(c) einem oder mehreren Carotinoiden und/oder Vitamin A, und
(d) einem oder mehreren natürlichen oder synthetischen Flavonoiden, Bioflavonoiden, Flavanolen oder Catechinen einschließlich Anthocyanen und deren Glykosiden,
sowie gegebenfalls
(v) für perorale, topische, parenterale oder rektale Verabreichungsformen pharmazeutisch geeignete Additive und/oder Trägerstoffe,
worin:
(α) das Mengenverhältnis der Komponenten (i), (ii), (iii) und (iv) zueinander der Bedingung genügt:
(i) : (ii) : (iii) : (iv) =
(0,05 - 2,0) Gew.-T. : (0,05 - 2,0) Gew.-T. : (bis zu 2,0) Gew.-T. : (0,05 - 1,0) Gew.-T.;
(β) bezogen auf eine Dosiseinheit, die von 50 bis 1000 mg jeder einzelnen Komponente (i), (ii) oder (iii) enthält, die Menge an Stabilisatorgemisch (iv) bis zu 500 mg beträgt; und
(γ) der Anteil an Ascorbinsäure oder dessen Salzen und Estern (a) im Stabilisatorgemisch (iv) pro Dosiseinheit bis zu 50 mg beträgt.

2. Stabilisiertes Arzneimittel gemäß Anspruch 1, **dadurch gekennzeichnet, daß** der Bestandteil (b) des Stabilisatorgemisches aus einer oder mehreren Verbindungen natürlichen oder synthetischen Ursprungs der Reihe α-, β-, γ-, δ- und ε-Tocopherol, all-rac-α-Tocopherol, Tocol, α-Tocopherolhydrochinon, α-Tocopherolchinon, oder deren Derivaten wie den Acetaten, Succinaten, Nicotinaten, Poly(oxyethylen)succinaten, Ubichinonen, Bovichinonen, Plastochinonen oder Menachinonen ausgewählt ist.

3. Stabilisiertes Arzneimittel gemäß Anspruch 1 oder 2, **dadurch gekennzeichnet, daß** der Bestandteil (c) des Stabilisatorgemisches aus einem oder mehreren Verbindungen sowie deren Derivaten natürlichen oder synthetischen Ursprungs der Reihe Canthaxanthin, Rhodoxanthin, Capsorubin, Zeaxanthin, α-, β-, γ-, δ-, ε- und/oder ζ-Carotin, Lycopin, Capsanthin, Cryptoxanthin, Crocetin, Lutein, Decapren-β-Carotin, Dodecapren-β-Carotin, Astaxanthin, Violaxanthin oder Bixin ausgewählt ist.

4. Stabilisiertes Arzneimittel gemäß einem der Ansprüche 1 bis 3, **dadurch gekennzeichnet, daß** der Bestandteil (d) aus einer oder mehreren Verbindungen sowie deren Derivaten wie Glukosiden natürlichen oder synthetischen Ursprungs der Reihe der Flavonoide bzw. Bioflavonoide oder Flavanole wie dem Chrysin, Apigenin, Fisetin, Kämpferol, Luteolin, Galangin, Gossypetin, Morin, Myricetin, Naringin, Quercetin, Robinetin, Anthocyaninen, Rutin, Hesperidin, Taxifolin, Catechin, Epicatechin, Epicatechingallat, Gallocatechin, Epigallocatechingallat, Tangeretin, Eriodictyol, Naringenin, Rutin, Troxerutin (Quercetinrutosid), Aesculin, Aesculetin, Skimmin, Umbelliferon, entsprechenden anderen Glykosiden wie Aesculosid oder Anthocyanosiden, Rutosiden wie dem Tri(hydroxyethyl)rutosid, Ruscogeninen, O-(β-Hydroxyethyl)rutosiden sowie natürlichen Extrakten aus Citrusfrüchten, Myrtillum Arten, Melilotus Arten, Hypericum Arten, oder anderen Bioflavonoide liefernden Pflanzenextrakten ausgewählt sind.

5. Stabilisiertes Arzneimittel gemäß einem der Ansprüche 1 bis 4, **dadurch gekennzeichnet, daß** als zusätzliches Stabilisierungsmittel bezüglich der Komponente (d) eine oder mehrere Verbindungen natürlichen oder synthetischen Ursprungs aus der Reihe der Polyphenole wie Kaffeesäureester, Kaffeesäureamide, Ethoxyquin, Carnosinsäure, Carnosol sowie Derivaten dieser Verbindungen, Auszügen aus Thea-Arten, Rosmarin-Arten oder anderen derartigen natürliche Phenolverbindungen liefernden Pflanzen enthalten sind.

6. Stabilisiertes Arzneimittel gemäß einem der Ansprüche 1 bis 7, **dadurch gekennzeichnet, daß** flüssige wäßrige oder Lipide enthaltende Zubereitungen als Solubilisatoren einen Lösungsvermittler aus der Reihe Polyoxyethylenglycerolfettsäureester in Form von Monolaurat, Monostearat, Monooleat, Triricinoleat oder Trihydroxystearat, Cremophor-Typen, Tegin-Typen, Phospholipiden wie z.B. Lecithinen ggf. neben organischen, pharmazeutisch verträglichen Lösungsmitteln wie Alkoholen in Form von Ethanol, Isopropanol, Butanol enthalten, wobei ein oder mehrere Wirkstoffe, einzeln oder in Kombination miteinander, insbesondere das N-Acetylcystein oder ein oder mehrere nichtsteroidale Entzündungshemmer(Antiphlogistika)/Analgetika in liposomaler feinstverteilter oder Targetform oder auch mikronisiert vorliegen können.

7. Stabilisiertes Arzneimittel gemäß einem der Ansprüche 1 bis 6, **dadurch gekennzeichnet, daß** es in Form von ggf. überzogenen, magensaftresistenten und/oder retardierten Kapseln, Komprimaten, Sublingual- oder Kautabletten, Kaugummi, Pastillen, Brausezubereitungen, Tabs, Granulaten, Pellets, Mikrokapseln, Trockensaft, Pulver, Injektions- und Infusionspräparaten, Suppositorien, Tropfen, Suspensionen, Lösungen, Dosieraerosolen, Verneblern, Zerstäubern, Sprays, Liposomenzubereitungen, transdermalen Zubereitungen, Pflastern, Pasten, Emulsionen, Cremes oder Gelen vorliegt.

8. Stabilisiertes Arzneimittel gemäß einem der Ansprüche 1 bis 6, **dadurch gekennzeichnet, daß** jeweils ein oder mehrere Wirkstoffe aus der Gruppe der jeweils stabilisierten cysteinylhaltigen Verbindung(en), dem Glutathion und den NSAID-Verbindung(en) in separater Form in den Arzneizubereitungen bzw. als getrennte Arzneiformen, beispielsweise als perorale feste Arzneiform, insbesondere in Form von Komprimaten oder Kapseln einerseits, und Ampullen andererseits in der Arzneimittelpackung nebeneinander vorliegen.

9. Stabilisiertes Arzneimittel gemäß einem der Ansprüche 1 bis 7, **dadurch gekennzeichnet, daß** es in Form einer Brausetablette, Brausegranulat oder Tabs vorliegt, wobei der Wirkstoff oder die Wirkstoffe (i) als Verbindung, die eine Cysteinylgruppe enthält, in Einzeldosierungen von 50 mg, 100 mg oder 200 mg oder der jeweils 2-, 3-, 4- oder 5-fachen Menge davon, insbesondere als N-Acetylcystein zusammen mit dem Stabilisatorgemisch aus den Komponenten (a), (b), (c) und (d) aus der Reihe Ascorbinsäureverbindung, Vitamin E (Tocopherole), Vitamin A (Carotinoide), und einem oder mehreren Flavonoiden wie Rutin, Aesculin, Catechin, pflanzlichen Polyphenolen wie Kaffeesäure und/oder bioflavonoidhaltigen Extrakt(en) aus Citrusfrüchten in Einzeldosierungen von jeweils 2,0 mg, 2,5 mg, 5,0 mg, 10 mg oder 20 mg oder der jeweils 2-, 3-, 4-, 5- oder 10-fachen Menge davon, in Kombination mit Glutathion (ii) in Einzeldosierungen von 50 mg, 100 mg, 200 mg oder 250 mg oder der jeweiligen 2-, 3- oder 4-fachen Menge davon, und in Kombination mit (iii) NSAID-Verbindungen, insbesondere mit Diclofenac jeweils in einer Menge von 50 mg, 100 mg oder 200 mg oder der jeweiligen 2-, 3-, 4-, oder 5-fachen Menge davon, zusammen mit üblichen Tablettier- und Granulierhilfsstoffen, sowie bei den Brausezubereitungen, mit Kohlendioxid entwickelnden organischen Säuren wie Zitronen- oder Weinsäure und Carbonaten wie Natriumhydrogencarbonat, Granulierhilfsstoffen, Aromastoffen, Süßungsmitteln wie Saccharin und/oder Zuckern wie Mannit, Sorbit und erforderlichenfalls üblichen Trägerstoffen vorliegen.

10. Stabilisiertes Arzneimittel gemäß einem der Ansprüche 1 bis 9, **dadurch gekennzeichnet, daß** es zusätzlich noch Spurenelemente, insbesondere Selen beispielsweise in einer Einzel- oder Tagesdosismenge von jeweils 0,01, 0,02, 0,05, 0,1, 0,2, 0,24, 0,4, 0,5 bis maximal 0,8 oder 1,0 mg als Selenit oder Selenat, z. B. als Natriumsalz enthält.

11. Stabilisiertes Arzneimittel gemäß einem der Ansprüche 1 bis 10 zur Behandlung von entzündlichen Erkrankungen der Bronchien und Lunge, von Otitis, Sinusitis, Laryngitis, Mukoviszidose, rheumatischen Störungen bzw. Entzündungen, Schmerzzuständen, Strahlenschäden, zur Detoxication, insbesondere von Arzneimittelschäden, zur Prophylaxe und Therapie von Carcinomen, ggf. in Kombination mit Cytostatika, zur Organperfusion oder Prophylaxe von Tranplantatabstoßungen.

12. Stabilisiertes Arzneimittel gemäß einem der Ansprüche 1 bis 10 zur therapeutischen oder prophylaktischen Behandlung von entzündlichen Erkrankungen der Atemwege und zur Unterdrückung von Organ- und Entzündungsschäden, insbesondere der Leber.

## Claims

1. Stabilised medicament made from the components
(i) compound(s) containing one or two cysteinyl group(s),
(ii) glutathione
(iii) one or more non-steroidal antiphlogistics (NSAID) and/or analgesic, selected from paracetamol, indomethacin, acemetacin, ibuprofen, ketoprofen, naproxen, sulindac, piroxicam, mefenaminic acid, phenylbutazone, phenazone, propyphenazone and metamizole, and their optionally existing pharmacologically active enantiomers or other optical isomers, and diclofenac,
(iv) a stabiliser mixture consisting of
(a) ascorbic acid (vitamin C) or its salts or esters,
(b) one or more tocopherols (vitamin E),
(c) one or more carotenoids and/or vitamin A, and
(d) one or more natural or synthetic flavonoids, bioflavonoids, flavanols or catechins including anthocyans and their glycosides,
and optionally
(v) additives and/or excipients pharmaceutically suitable for peroral, topical, parenteral or rectal forms of administration,
wherein:
(α) the quantity ratio of the components (i), (ii), (iii) and (iv) with respect to one another satisfies the condition:
(i) : (ii) : (iii) : (iv) =
(0.05 - 2.0) parts by weight : (0.05 - 2.0) parts by weight : (up to 2.0) parts by weight: (0.05 - 1.0) parts by weight;
(β) based on a dose unit, which contains from 50 to 1,000 mg of each individual component (i), (ii) or (iii), the quantity of stabiliser mixture (iv) is up to 500 mg; and
(γ) the proportion of ascorbic acid or its salts and esters (a) in the stabiliser mixture (iv) per dose unit is up to 50 mg.

2. Stabilised medicament according to claim 1, **characterised in that** the constituent (b) of the stabiliser mixture is selected from one or more compounds of natural or synthetic origin of the series α-tocopherol, β-tocopherol, γ-tocopherol, δ-tocopherol and ε-tocopherol, all-rac-α-tocopherol, tocol, α-tocopherol hydroquinone, α-tocopherol quinone, or their derivatives, such as the acetates, succinates, nicotinates, poly(oxyethylene) succinates, ubiquinones, boviquinones, plastoquinones or menaquinones.

3. Stabilised medicament according to claim 1 or 2, **characterised in that** the constituent (c) of the stabiliser mixture is selected from one or more compounds and their derivatives of natural or synthetic origin of the series canthaxanthin, rhodoxanthin, capsorubin, zeaxanthin, α-carotene, β-carotene, γ-carotene, δ-carotene, ε-carotene and/or ζ-carotene, lycopin, capsanthin, cryptoxanthin, crocetin, lutein, decapren-β-carotene, dodecapren-β-carotene, astaxanthin, violaxanthin or bixin.

4. Stabilised medicament according to one of claims 1 to 3, **characterised in that** the constituent (d) are selected from one or more compounds and their derivatives, such as glucosides of natural or synthetic origin of the series of flavonoids or bioflavonoids or flavanols, such as chrysin, apigenin, fisetin, camphor oil, luteolin, galangin, gossypetin, morin, myricetin, naringin, quercetin, robinetin, anthocyanins, rutin, hesperidin, taxifolin, catechin, epicatechin, epicatechin gallate, gallocatechin, epigallocatechin gallate, tangeretin, eriodictyol, naringenin, rutin, troxerutin (quercetin rutoside), aesculin, aesculetin, skimmin, umbelliferone, corresponding other glycosides, such as aesculoside or anthocyanosides, rutosides, such as the tri(hydroxyethyl) rutoside, ruscogenins, O-(β-hydroxyethyl) rutosides and natural extracts of citrus fruits, myrtillum species, melilotus species, hypericum species, or other plant extracts providing bioflavonoids.

5. Stabilised medicament according to one of claims 1 to 4, **characterised in that** as additional stabilising agent with regard to component (d), one or more compounds of natural or synthetic origin from the series of polyphenols, such as caffeic acid esters, caffeic acid amides, ethoxyquin, carnosinic acid, carnosol and derivatives of these compounds, extracts from thea species, rosemary species or other plants providing such natural phenol compounds, are present.

6. Stabilised medicament according to one of claims 1 to 5, **characterised in that** liquid aqueous lipid-containing formulations contain as solubilisers a solubility promoter from the series polyoxyethylene glycerol fatty acid esters in the form of monolaurate, monostearate, monooleate, triricinoleate or trihydroxystearate, cremophor types, tegin types, phospholipids, such as for example lecithins optionally in addition to organic, pharmaceutically acceptable solvents, such as alcohols in the form of ethanol, isopropanol, butanol, wherein one or more active ingredients may be present, individually or in combination with one another, in particular the N-acetylcystein or one or more non-steroidal anti-inflammatories (antiphlogistics)/analgesics in liposomal very finely divided or target form or also micronised.

7. Stabilised medicament according to one of claims 1 to 6, **characterised in that** it is present in the form of optionally coated, gastric juice-resistant and/or delayed-release capsules, compressed tablets, sublingual or chewable tablets, chewing gum, pastilles, effervescent formulations, tablets, granules, pellets, microcapsules, dry suspension, powders, injection and infusion preparations, suppositories, drops, suspensions, solutions, metered aerosols, nebulisers, atomisers, sprays, liposome formulations, transdermal formulations, plasters, pastes, emulsions, creams or gels.

8. Stabilised medicament according to one of claims 1 to 6, **characterised in that** in each case one or more active ingredients from the group of the particular stabilised cysteinyl-containing compound(s), glutathione and the NSAID compound(s) are present in separate form in the medicament formulations or as separate medicament forms, for example as peroral solid medicament form, in particular in the form of compressed tablets or capsules on the one hand, and ampoules on the other hand, next to one another in the medicament package.

9. Stabilised medicament according to one of claims 1 to 7, **characterised in that** it is present in the form of an effervescent tablet, effervescent granules or tablets, wherein the active ingredient or the active ingredients (i) are present as a compound which contains a cysteinyl group, in individual doses of 50 mg, 100 mg or 200 mg or in each case 2, 3, 4 or 5 times the quantity thereof, in particular as N-acetylcystein together with the stabiliser mixture of the components (a), (b), (c) and (d) from the series ascorbic acid compound, vitamin E (tocopherols), vitamin A (carotenoids), and one or more flavonoids, such as rutin, aesculin, catechin, vegetable polyphenols, such as caffeic acid and/or bioflavonoid-containing extract(s) from citrus fruits in individual doses of in each case 2.0 mg, 2.5 mg, 5.0 mg, 10 mg or 20 mg or in each case 2, 3, 4, 5 or 10 times the quantity thereof, in combination with glutathione (ii) in individual doses of 50 mg, 100 mg, 200 mg or 250 mg or in each case 2, 3 or 4 times the quantity thereof, and in combination with (iii) NSAID compounds, in particular with diclofenac in each case in a quantity of 50 mg, 100 mg or 200 mg or in each case 2, 3, 4, or 5 times the quantity thereof, together with conventional tabletting and granulation auxiliaries, and for the effervescent formulations, with organic acids which develop carbon dioxide, such as citric acid or tartaric acid and carbonates, such as sodium hydrogen carbonate, granulation auxiliaries, flavouring agents, sweeteners, such as saccharin and/or sugars, such as mannitol, sorbitol and if required conventional excipients.

10. Stabilised medicament according to one of claims 1 to 9, **characterised in that** it additionally contains trace elements, in particular selenium, for example in an individual or daily dose quantity of in each case 0.01, 0.02, 0.05, 0.1, 0.2, 0.24, 0.4, 0.5 up to a maximum 0.8 or 1.0 mg as selenite or selenate, for example as sodium salt.

11. Stabilised medicament according to one of claims 1 to 10 for treating inflammatory diseases of the bronchial tubes and lungs, otitis, sinusitis, laryngitis, mucoviscidosis, rheumatic disturbances or inflammations, painful states, radiation damage, for the detoxification, in particular of medicament damage, for the prophylaxis and treatment of carcinomas, optionally in combination with cytostatic agents, for organ perfusion or prophylaxis of transplant rejections.

12. Stabilised medicament according to one of claims 1 to 10 for the therapeutic or prophylactic treatment of inflammatory diseases of the respiratory passages and for suppressing organ lesions and inflammation lesions, in particular of the liver.

## Revendications

1. Médicament stabilisé constitué des composants suivants :
(i) un composé ou des composés contenant un ou deux groupes cystéinyle,
(ii) du glutathion,
(iii) un ou plusieurs anti-inflammatoires non stéroïdiens (NSAIDs) et / ou analgésiques, choisis parmi le paracétamol, l'indométacine, l'acémétacine, l'ibuprofène, le kétoprofène, le naproxène, le sulindac, le piroxicame, l'acide méfénamique, la phénylbutazone, la phénazone, la propyphénazone, et le métamizol, ainsi que leurs énantiomères existants, actifs sur le plan pharmacologique et d'autres isomères optiques, et le diclofenac,
(iv) un mélange de stabilisants, constitué par
(a) l'acide ascorbique (vitamine C), ses sels ou ses esters,
(b) un ou plusieurs tocophérols (vitamine E),
(c) un ou plusieurs caroténoïdes et / ou la vitamine A et
(d) un ou plusieurs composés naturels ou synthétiques du type flavonoides, bioflavonoïdes, flavanolènes ou catéchines, y compris les anthocyanes et leurs glycosides,
ainsi que, le cas échéant :
(v) des additifs et / ou des excipients convenant à des formes galéniques adaptés à une administration perorale, topique, parentérale ou rectale,
où:
(α) les rapports pondéraux des composés (i), (ii), (iii) et (iv) satisfont aux conditions :
(i) : (ii) : (iii) : (iv) =
(0,05 - 2,0) parties en poids : (0,05 - 2,0) parties en poids : (jusqu'à 2,0) parties en poids : (0,5 - 1,0) parties en poids ;
(β) chaque unité d'administration contient de 50 à 1000 mg de chaque composant individuel (i), (ii) ou (iii) et un mélange de stabilisants (iv) allant jusqu'à 500 mg ; et
(y) la fraction d'acide ascorbique, ou des sels et des esters (a) de celui-ci dans le mélange des stabilisants (iv) va jusqu'à 50 mg, par unité d'administration.

2. Médicament stabilisé selon la revendication 1, **caractérisé en ce que** la fraction (b) du mélange de stabilisants est constituée par un ou plusieurs composés naturels ou synthétiques, choisi ou choisis dans le groupe constitué par l'α-, le β-, le γ-, le δ- et l'ε-tocophérol, l'all-rac-α-tocophérol, le tocol, l'α-tocophérolhydroquinone, l'α-tocophérolquinone ou leurs dérivés tels que les acétates, les succinates, les nicotinates, les poly(oxyéthylène)succinates, les ubiquinones, les boviquinones, les plastoquinones, les ménaquinones, etc.

3. Médicament stabilisé selon la revendication 1 ou la revendication 2, **caractérisé en ce que** la fraction (c) du mélange de stabilisants est constituée par un ou plusieurs composés ainsi que par leurs dérivés naturels ou synthétiques, choisi ou choisis dans le groupe constitué par la canthaxanthine, la rhodoxanthine, la capsorubine, la zéaxanthine, l'a-, la β-, la γ-, la δ-, l'ε- et / ou la ζ-carotine, la lycopine, la capsanthine, la cryptoxanthine, la crocétine, la lutéine, la décaprène-β-carotine, la dodécaprène-β-carotine, l'astaxanthine, la violaxanthine et la bixine.

4. Médicament stabilisé selon l'une des revendications 1 à 3, **caractérisé en ce que** la fraction (d) est constituée par un ou plusieurs composés ainsi que par leurs dérivés tels que leurs glucosides naturels ou synthétiques, choisi ou choisis dans le groupe constitué par les flavonoïdes, les bioflavonoïdes ou les flavanoles, tels que la chrysine, l'apigénine, la fisetine, le camphérol, la lutéoline, la galangine, la gossypétine, la morine, la myricétine, la naringine, la quercétine, la robinétine, l'anthocyanine, la rutine, l'hespéridine, la taxifoline, la catéchine, l'épicatéchine, le gallate d'épicatéchine, la gallocatéchine, le gallate de l'épigallocatéchine, la tangérétine, l'ériodictoyle, la naringénine, la rutine, la troxérutine (quercétinrutoside), l'aesculine, l'aesculétine, la skimmine, l'umbelliférone, d'autres glycosides correspondants tels que l'aesculoside ou les anthocyanosides, les rutosides tels que le tri(hydroxyéthyl)rutoside, les ruscogénines, les O-(β-hydroxyéthyl)rutosides, ainsi que les extraits naturels d'agrumes, d'espèces des genre Myrtillum, Melilotus et Hypericum ou d'autres extraits de plantes contenant des bioflavonoïdes.

5. Médicament stabilisé selon l'une des revendications 1 à 4, **caractérisé en ce que**, comme stabilisants additionnels appartenant au groupe (d), on peut utiliser un ou plusieurs composés naturels ou synthétiques choisis dans le groupe de polyphénols, tels que les esters de l'acide caféique, l'amide de l'acide caféique, l'éthoxyquine, l'acide carnosique, le carnosol, ainsi que les dérivés de ces composés, des extraits de plantes des genres Thea et Rosmarinus et également d'autres plantes fournissant des composés polyphénols naturels similaires.

6. Médicament stabilisé selon l'une des revendications 1 à 5, **caractérisé en ce que** la préparation liquide aqueuse ou lipidique contient, en tant que solubilisant, un agent favorisant la solubilité choisi dans le groupe constitué par des esters d'acides gras de polyoxyéthylène-glycérols sous la forme du monolaurate, du monostéarate, du monooléate, du triricinoléate, ou du trihydroxystéarate, des composés du type cremophor et tegin, des phospholipides tels que, par exemple, la lécithine, le cas échéant, à côté de solvants organiques acceptables sur le plan pharmaceutique, tels que des alcools (en particulier, éthanol, isopropanol, butanol), où un composé actif ou une combinaison de composés actifs, en particulier la N-acétylcystéine, ou un ou plusieurs anti-inflammatoires non stéroïdiens (antiphlogistiques) / analgésiques peut être présent ou peuvent être présents en forme liposomale finement répartie, ou dans une forme galénique à cible définie ou encore sous une forme micronisée.

7. Médicament stabilisé selon l'une des revendications 1 à 6, **caractérisé en ce qu'**il est fourni sous une forme enrobée, sous une forme résistante aux sucs gastriques, et / ou sous la forme de capsules à effet retardé, de comprimés, de comprimés sublinguaux ou de comprimés à mâcher, de gomme à mâcher, de pastilles, de préparations effervescentes, de tabs, de granulés fins ou plus grossiers, de microcapsules, d'extraits secs, de poudres, de préparations pour injection, de préparations pour perfusion, de suppositoires, de gouttes, de suspensions, de solutions, d'aérosols doseurs, de nébuliseurs, d'atomiseurs, de sprays, de préparations de liposomes, de préparations transdermiques, d'autoadhésifs, de pâtes, d'émulsions, de crèmes ou de gels.

8. Médicament stabilisé selon l'une des revendications 1 à 6, **caractérisé en ce qu'**une substance active ou plusieurs substances actives choisie ou choisies dans le groupe constitué par des composés stabilisés contenant un groupe cystéinyle, le glutathion et un ou des composés NSAID est ou sont conditionnées côte-à-côte en tant que préparations pharmaceutiques distinctes ou en tant que formes galéniques distinctes, avec par exemple, d'une part une forme solide convenant à une administration perorale, en particulier des comprimés ou des capsules et, d'autre part, des d'ampoules.

9. Médicament stabilisé selon l'une des revendications 1 à 7, **caractérisé en ce qu'**il se présente sous la forme de comprimés effervescents, de granulés effervescents ou de tablettes, où la substance active / les substances actives est / sont (i) un composé / des composés contenant un groupe cystéinyle, présent / présents, dans chaque unité d'administration, à raison de 50 mg, de 100 mg ou de 200 mg ou d'une quantité correspondant à un multiple par 2, 3, 4 ou 5 de ces grandeurs, en particulier sous la forme de N-acétylcystéine ; en combinaison avec un mélange des stabilisants constitué par (a), (b), (c) et (d), choisis plus particulièrement dans le groupe constitué par les composés de l'acide ascorbique, la vitamine E (tocophérols), la vitamine A (caroténoïdes), un ou des flavonoïdes tels que la rutine, l'aesculine et la catéchine, des polyphénols d'origine végétale tels que l'acide caféique et / ou des extraits contenant des bioflavonoïdes d'agrumes, à raison de 2,0 mg, de 2,5 mg, de 5,0 mg, de 10 mg ou de 20 mg ou d'une quantité correspondant à un multiple par 2, 3, 4, 5 ou 10 de ces grandeurs (par unité d'administration) ; en combinaison avec du glutathion (ii) à raison de 50 mg, de 100 mg, de 200 mg, ou de 250 mg ou d'une quantité correspondant à un multiple par 2, 3, ou 4 de ces grandeurs (par unité d'administration) ; en combinaison avec des composés NSAIDs (iii), en particulier avec du diclofenac, à raison de 50 mg, de 100 mg ou de 200 mg ou d'une quantité correspondant à un multiple par 2, 3, 4 ou 5 de ces grandeurs ; et en combinaison avec des substances habituelles utilisées pour la fabrication de comprimés ou de granulés et, en particulier, dans le cas de préparations effervescentes, des acides organiques capables de provoquer la formation de gaz carbonique tels que l'acide citrique ou l'acide tartrique et des carbonates tels que l'hydrogénocarbonate de sodium, ainsi que des adjuvants de granulation, des composés aromatiques, des édulcorants tels que la saccharine et / ou des sucres tels que le mannitol, le sorbitol et, le cas échéant, les excipients habituels.

10. Médicament stabilisé selon l'une des revendications 1 à 9, **caractérisé en ce qu'**il contient en plus, des oligo-éléments, en particulier et par exemple du sélénium, présent dans l'unité d'administration ou dans la dose journalière à raison de 0,01, de 0,02, de 0,05, de 0,1, de 0,2, de 0,24, de 0,4, de 0,5 ou, au maximum, de 0,8 ou de 1,0 mg, cette quantité étant exprimée en sélénite ou en sélénate, par exemple en tant que sel de sodium.

11. Médicament stabilisé selon l'une des revendications 1 à 10, pour le traitement de maladies inflammatoires des bronches et des poumons, d'otites, de sinusites, de laryngites, de la mucoviscidose, de maladies rhumatiques et, en particulier, inflammatoires, de douleurs, des dommages causés par les radiations, pour faciliter les désintoxications, en particulier celles associées à la prise de médicaments, pour le traitement préventif et thérapeutique de carcinomes, le cas échéant en combinaison avec des cytostatiques, pour la perfusion d'organes ou la prévention de rejets de transplants.

12. Médicament stabilisé selon l'une des revendications 1 à 10, pour le traitement thérapeutique ou prophylactique de maladies inflammatoires des voies respiratoires et pour diminuer les atteintes causées à des organes et les atteintes inflammatoires, en particulier du foie.
